# EUROPEAN PATENT APPLICATION

(11) **EP 3 858 252 A2**
(43) Date of publication of application: **04.08.2021**
(21) Application number: 21153771.7
(22) Date of filing: 27.01.2021
(51) Int. Cl.: A61B 10/00, G01N 1/28, G02B 21/34, A61B 10/02

(54) **NON-DESTRUCTIVE BIOPSY TISSUE IMAGING AND DIAGNOSIS**

(30) Priority: 28.01.2020 US 202062966771 P; 23.11.2020 US 202017102374
(71) Applicant: Covidien LP, Mansfield, MA 02048 (US)
(72) Inventor: GRIM, Kasey, Boulder, CO Colorado 80301 (US); SARTOR, Joe D., Longmont, CO Colorado 80504 (US); WARREN, Jamie, Boulder, CO Colorado 80301 (US)
(74) Representative: Maschio, Antonio

(57) **Abstract**

A method and system of imaging a tissue sample including receiving a biopsy device in a first channel of a microscopy cassette, injecting a first fluid into the biopsy device with a fluid to direct the tissue sample into a capture tube, injecting a second fluid into a second channel of the microscopy cassette, receiving the tissue sample in a removable insert, draining the first and second fluids from the removable insert, and acquiring an image of the tissue sample.

## Description

### CROSS REFERENCE TO RELATED APPLICATION

The present application claims the benefit of and priority to U.S. Provisional Application Serial No. 62/966,771, filed on January 28, 2020 the entire contents of which are incorporated herein by reference.

### FIELD

This disclosure relates to the field of imaging, and particularly to an imaging system including a biopsy specimen holder and a cassette enabling real-time microscopy while ensuring consistent and safe handling of the specimen for further histological examination.

### BACKGROUND

Diagnostic biopsies are commonly performed under distant navigation. For example, lung biopsies and gastro-intestinal biopsies are commonly performed after insertion and navigation of an endoscope or catheter to a location proximate the lesion or tumor. Similarly, ultrasound guided breast biopsy can employ various navigation and imaging techniques to place the biopsy device proximate the suspected tumor. Typically, physicians performing the biopsy take multiple samples to provide additional assurance that the correct tissue has in fact been sampled and that a diagnosis can be rendered by a pathologist. Each additional sample increases the cost to process it and incurs greater risk to the patient in bleeding and adverse events related to collection of the sample. Further traditional pathology analysis can take days or weeks to receive from the laboratory and further time for the attending physician to provide to the patient. While there are instances where a pathologist may be available to make gross pathology examinations, having a pathologist present greatly increases the cost of the procedures and is typically only provided in procedures of greatest risk or where there is great benefit to the patient.

Spectrally-encoded confocal microscopy (SECM) is a high-speed reflectance confocal microscopy (RCM) technology that has a potential to rapidly image the entire biopsy specimen at sub-cellular resolution. SECM techniques are part of a system of Rapid On-site Evaluation (ROSE) techniques that may be employed at the time of the biopsy to confirm margins on treatment, ensure sufficiency of the biopsy sample. While these techniques are very good, they are typically employed in addition and prior to traditional histological examination.

The more times that a biological sample is handled, and the more times that it is prepared for analysis, the greater the risk of damage to the sample, and potential confusion as to the sample's provenance. Typical procedures for histological examination require that the sample is either frozen or placed in a fixative such as paraffin for microtome processing.

Prior to fixation and microtome processes, the sample is typically placed in solution of formalin. Formalin helps to stop enzyme activity, kill microorganisms, and harden the specimen while maintaining the molecular structure of the sample to enable staining of the microtome (sections of the specimen) for analysis. Additional steps including grossing (reducing the size of the specimen), embedding, and others may also be required depending on the sample.

As can be appreciated, accurate and consistent tracking of the sample through so many steps, is imperative to an accurate diagnosis and improvements in such technologies is always desired.

### SUMMARY

This disclosure is directed to imaging system that allows immediate tissue analysis at the time of biopsy without requiring a pathologist to be present. The system enables automatic extraction of tissue from the biopsy device, evaluation of the sample, and preservation of the sample constrained in a removable component to facilitate both fixation and further histopathologic diagnosis.

One aspect of the disclosure is directed to a microscopy cassette including: a first channel for receiving a biopsy device, a second channel in communication with a fluid source. The microscopy cassette also includes a capture tube in communication with the first channel and the second channel. The microscopy cassette also includes a recess configured to receive a removable insert and in fluid communication with the capture tube. The microscopy cassette also includes a glass lid covering the recess

Implementations of aspects of the disclosure may include one or more of the following features. The microscopy cassette may further include a reservoir in fluid communication with the recess, a vacuum connection, where application of suction by the vacuum connection promotes movement of fluid from the recess to the reservoir, or a seal on the first channel configured to accept the biopsy device and prevent fluid from exiting the first channel. The microscopy cassette may further include a removable insert received in the recess. The removable insert may be configured to receive a biopsy sample from the biopsy device. The fluid from the biopsy device and from the second channel carries a tissue sample through the capture tube and into the removable insert.

A further aspect of the disclosure is directed to a removable insert including: a top region, a bottom region, two ends and two angled side walls; an opening formed in one of the ends configured to receive a tissue sample. The removable also includes a mesh material formed on the bottom region and traversing an opening in the bottom region, the mesh material being liquid permeable but sized to retain a tissue sample received thereon. The removable also includes a lid operably connected to one of the angled side walls, and movable from an open position to a closed position on the top region.

Implementations of this aspect of the disclosure may include one or more of the following features. The removable insert may further include a fluid permeable material on the lid permitting fluid to enter the removable insert when the lid is in the closed position, an end portion formed on the lid and covering the opening when the lid is in the closed position. The top region, bottom region, two ends and two angled side walls are configured for receipt into a microscopy cassette. The opening of the removable insert may be formed in one of the ends is configured to receive an end of a capture tube of the microscopy cassette.

Yet another aspect of the disclosure is directed to a method of imaging a tissue sample including receiving a biopsy device in a first channel of a microscopy cassette. The method of imaging also includes injecting a first fluid into the biopsy device with a fluid to direct the tissue sample into a capture tube. The method of imaging also includes injecting a second fluid into a second channel of the microscopy cassette. The method of imaging also includes receiving the tissue sample in a removable insert. The method of imaging also includes draining the first and second fluids from the removable insert. The method of imaging also includes acquiring an image of the tissue sample.

Implementations of this aspect of the disclosure may include one or more of the following features. The method may further include closing a glass lid on the microscopy cassette, translating the microscopy cassette and acquiring a plurality of images, or applying a vacuum to assist in draining the first and second fluids from the removable insert. The method may further include transmitting the acquired image to a display, transmitting the acquired image to an application operating on a workstation where an application processes the image to make an initial pathology determination. The method may further include transmitting the acquired image to a laboratory for pathological examination. The method may further employ a robot to insert the biopsy device into the first channel of the microscopy cassette. Implementations of the described techniques may include hardware, a method or process, or computer software on a computer-accessible medium, including software, firmware, hardware, or a combination of them installed on the system that in operation causes or cause the system to perform the actions. One or more computer programs and applications can be configured to perform particular operations or actions by virtue of including instructions that, when executed by data processing apparatus, cause the apparatus to perform the actions.

### BRIEF DESCRIPTION OF THE DRAWINGS

Various exemplary embodiments are illustrated in the accompanying figures. It will be appreciated that for simplicity and clarity of the illustration, elements shown in the figures referenced below are not necessarily drawn to scale. Also, where considered appropriate, reference numerals may be repeated among the figures to indicate like, corresponding or analogous elements. The figures are listed below.
Fig. 1 is a schematic view of a spectrally-encoded confocal microscopy (SECM) system;
Fig. 2 is schematic of a cassette in accordance with the disclosure for use with the SECM system of Fig. 1;
Fig. 3 is a top perspective view of a removable insert in accordance with the disclosure;
Fig. 4 a front perspective view of the removable insert including a closeable lid in the open position; and
Fig. 5 a front perspective view of the removable insert including a closeable lid in the closed position.
Fig. 6 is a perspective view of a robotic arm in accordance with the disclosure;
Fig. 7 is a flow diagram of a method in accordance with the disclosure; and
Fig. 8 is a schematic view of a robotic biopsy and imaging system in accordance with the disclosure.

### DETAILED DESCRIPTION

This disclosure is directed to imaging system that allows immediate tissue analysis at the time of biopsy without requiring a pathologist to be present. The system enables extraction of tissue from the biopsy device, evaluation of the sample, and preservation of the sample constrained in a removable component to facilitate both fixation and further histopathologic diagnosis. These features improve the efficiency of the biopsy process by enabling near immediate verification that the tissue collected in the biopsy device was from a lesion or tumor and that the specimen is of diagnostic quality. The system and its use also benefit the patient by providing immediate gross findings all while the patient is present with the attending physician rather than days or weeks. The systems and methods of the disclosure can be used to analyze a variety of tissue types including lung, gynecological, and gastro-intestinal tissue. It will improve patient care and access to diagnosis.

In accordance with one aspect of the disclosure tissue is extracted from a biopsy needle and prepared for visualization. Microscopic images of the tissue are acquired using an SECM or other relevant techniques all while the sample is retained in a re-closable container to preserve the sample for further pathology analysis. The acquired images from the microscopy can be immediately analyzed by the attending physician to enable ROSE techniques to be employed even without a pathologist in attendance or electronically shared with the pathologist for analysis.

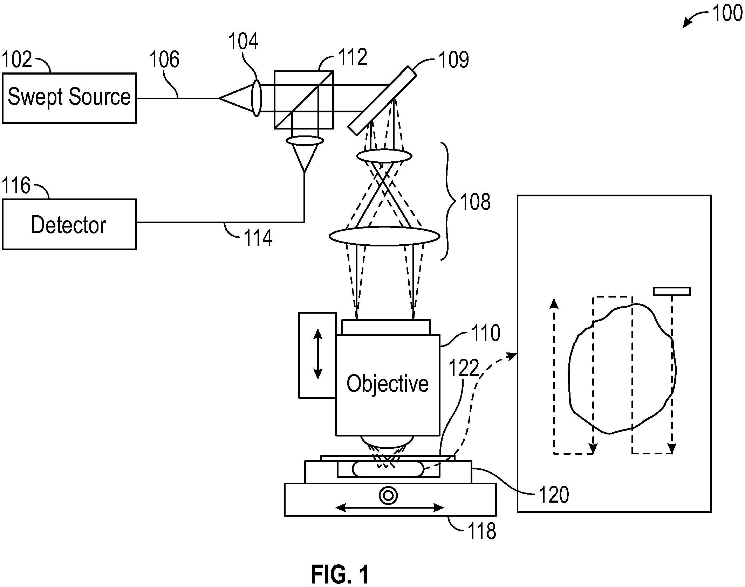

Fig. 1 depicts a SECM system 100. The SECM system 100 includes a light source 102 out putting a desired wavelength of light for imaging. In one example, light source is a wavelength-swept source with a repetition rate of 5 kHz, a central wavelength of 1320 nm, and a bandwidth of 70 nm. A collimation lens 104 receives the light from the light source 102 via a single mode fiber 106. A transmission grating 109 diffracts the light emanating from the light source 102. Relay optics 108 magnify the diffracted light and delivers it to an objective lens 110 where the light is focused on the tissue sample. The objective lens 110 may be a water immersion objective lens. Light is reflected from the tissue sample and collected by the objective lens 110 and travels back to the beam splitter 112. Half of the light is reflected at the beam splitter 112, coupled into a multi-mode fiber 114, and directed to a photo detector 116 to acquire images of the tissue sample. Large-area SECM images of the tissue may be acquired by raster-scanning the tissue sample using a two-axis motorized translation stage 118.

To image tissue samples, the sample must be placed in the specimen holder 120. A glass cover 122 may be placed over the tissue sample and where appropriate water may provide an interface between the objective lens 110, the glass cover 122 and the tissue sample. The tissue sample may be treated with, for example, and acetic acid solution to enhance contrast for imaging. To perform SECM imaging, of a sample about 10-15 minutes may be required in order to allow time for the translation of the translation stage 118 such that the entire tissue sample is imaged. Though SECM is described herein in detail, the disclosure is not so limited and the methods and devices described herein may be used in conjunction with other forms of imaging including traditional confocal, scanning and slit microscopy as well as others.

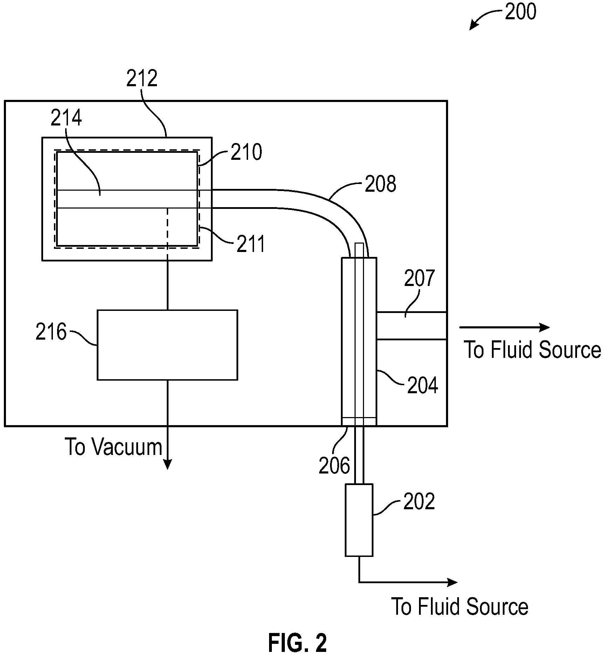

Fig. 2 depicts a cassette 200 in accordance with the disclosure. The cassette 200 is designed to receive a biopsy device 202 within a first channel 204. The biopsy device 202 may be a side-bite biopsy needle, or an end biting needle. A seal 206 prevents tissue or fluids, as will be described herein from exiting the cassette 200. The biopsy device 202 may be connected to a fluid source, which may be a syringe or a pressurized air or water source, via a luer-lock or other connection typical of biopsy devices. The fluid source can be used to apply differential pressure to the biopsy device 202 such that a tissue sample that has been retrieved from a patient and is located within the biopsy device is ejected out of the biopsy device and into a capture tube 208 formed in the cassette 200. A second channel 207 is connected to a fluid source, which may be the same or different from that which connects to the biopsy device 202. The fluid that is received from the second fluid source propels the tissue sample though the capture tube 208 and into a removable insert 210. The removable insert 210 is removable from the cassette 200 and has its own closeable lid. The removable insert 210 rests in a recess 211 shaped to receive and support the removable insert 210. The removable insert 210 is described in greater detail below. The cassette 200 includes a glass lid 212 that can be opened and closed and allows for imaging of a tissue sample using an SECM or other microscopy imaging device in accordance with the disclosure. An opening 214 formed in the bottom of the removable insert 210. This opening allows fluid from the biopsy device 202 or the second fluid channel to flow through the cassette 200 and be captured in a reservoir 216. The reservoir may be connected to a vacuum source to promote flow of fluid from the removable insert 210 to the reservoir 216. The collection of the fluid within the cassette 200 ensures that neither the fluid nor any biological material from the tissue sample escapes from the cassette 200 and ensures that the microscopy system (e.g., SECM 100) is maintained in a clean, possibly sterile condition preventing contamination of the tissue sample, the equipment, and the clinicians operating the equipment. The seal 206 associated with the first channel 204 helps to ensure that no fluid or biological matter escapes from the cassette 200 in that direction. A similar seal (not shown) may be formed on the second channel 206 as well.

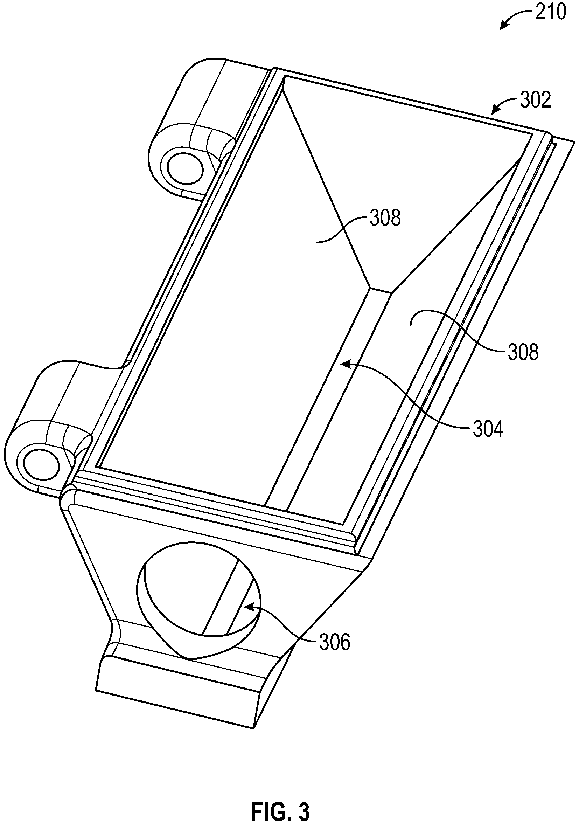

With reference to Fig. 1, the cassette 200 of Fig. 2 replaces the specimen holder 120 and is placed on the translation stage 118 for imaging. The removable insert 210, which is described in greater detail with reference to Figs. 3-5, encloses the tissue sample, and can be removed from the cassette 200 and placed in a formalin solution for further histological processing.

The removable insert 210 is depicted in Fig. 3 without its closeable lid. The removable insert 210 includes has a frusto-conical or trapezoidal shape, with a larger opening on a top region 302 as compared to a bottom region 304. This trapezoidal shape matches a similar shape of a recess 211 formed in the cassette 200 for receiving the removable insert 210. A first opening 306 is formed in one end of the removable insert 210. The first opening 306 mates with the capture tube 208, and a seal may be formed between the removable insert 210 and the capture tube 208 to prevent leaking of fluid at their mating point. The bottom region 304 is open and may be covered with a fine mesh material. The mesh material allows the fluid used to eject the tissue sample from the biopsy device 202 and from the second channel 206 to force the tissue sample through the capture tube 208 to drain from the removable insert 210 and be drawn to the reservoir 216. Side walls 308 are angled towards the opening in the bottom region and center the tissue sample in the removable insert 210.

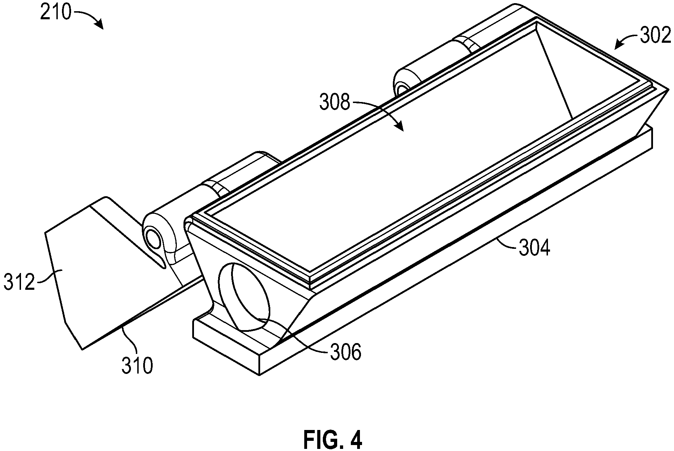

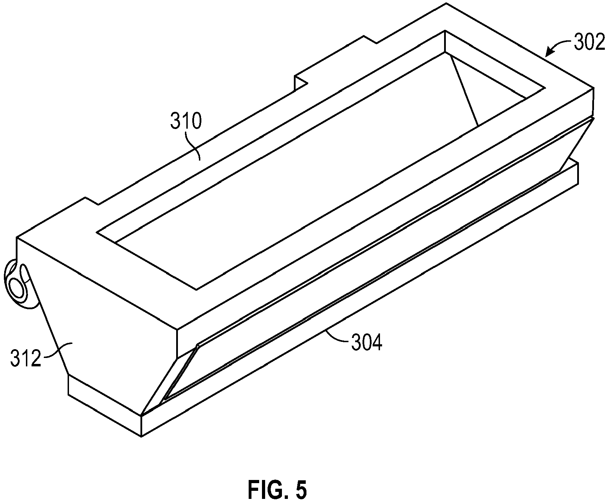

Fig. 4 depicts the removable insert 210 with its lid 310 in the open position. The lid 310 on the removable insert 210 may include a permeable material which allows preparation fluids to reach the tissue samples without allowing any biological material to pass through the permeable material. The permeable material may be for example a fine mesh material, similar to what is formed in the bottom region 304, allowing fluid to flow out of the removable insert 210 but not the tissue sample. In Fig. 5 the lid 310 is in the closed position, an end portion 312 of the lid 310 covers the first opening 306 sealing that opening and preventing a tissue sample contained therein from escaping.

The use of the cassette 200 and removable insert 210 for ROSE microscopy process and subsequent histological examination can now be described. Following acquisition of a biopsy sample from a patient, the biopsy device 200 is inserted through seal 206 and into capture tube 208. The seal 206, which may be a valve or a clamp creates a water-tight seal around the biopsy device 202.

Biopsy device 202 is inserted the first channel 204, past the second channel 207 and into the capture tube 208. A fluid, which may be air or a liquid is dispensed through the biopsy device 202 to expel the tissue sample into the capture tube 208. Fluid is also passed through the second channel 207, past a tip of the biopsy device 202 to carry tissue sample through the opening 306 in the removable insert 210 that mates with the capture tube 208.

The first and second channels 204, 207 may be flushed with fluid using syringes that can be connected thereto or automated pumps. The cassette 200 may also attach to microscopy system 100, which may include automated fluid air connections to the first and second channels 204, 207. The fluids, if liquid may include saline, vinegar, acetic acid or a hematoxylin stain to improve nuclear contrast of the tissue sample.

By application of pressure by the fluid, the tissue sample flows out of the biopsy device 202 and through capture tube 208. The tissue sample and fluid and drop into removable insert 210. The tissue sample then slides down the angled side walls 308 and lands on the mesh material covering the opening in the bottom region 304. The fluid is drains out of the removable insert 210 and is collected by the reservoir 216, as described above.

The end of the capture tube 208 is above the bottom region 304 of the removable insert 210, allowing tissue sample to drop a short distance from end of the capture tube 208 into the removable insert 210 and is supported by the mesh material. The angled side walls 308 may include a non-stick coating or lubricious material to promote movement of the tissue sample to the bottom region 304.

As noted above, the mesh material may be selected to allow fluid to pass through but catch tissue samples. The mesh width may be based on typical size of tissue samples, to allow them to fall into straight line without the tissue samples stacking or resting on walls. This enables efficient scanning with imaging system 100 through a transparent glass lid 212 of the cassette 200.

The fluid drains out of removable insert 210 and into the reservoir 216 within the cassette 200. The reservoir 216 stores the drained fluid and prevents leakage of the fluid out of the cassette 200. Additionally or alternatively, the cassette 210 may include a wicking material (not shown) that improves fluid flow through mesh or a connection to a vacuum source to provide suction for the same purpose.

After receipt of the tissue sample into the removable insert 210, the biopsy device 200 may be removed from the cassette. As noted above seal 206, which may be a one-way valve, prevents fluid from leaking from the cassette 200 and the first channel 204.

Following deposit of the tissue sample into the removable insert 210 the glass lid 212 is closed. The glass lid 212 latches shut and forms a seal with the removable insert 210 by pressing the upper region 302 and specifically a lip 314 of the removable insert against a gasket (not shown) formed on the glass lid 212.

The entire cassette 200 with removable insert 210 is placed in imaging device 100. The cassette 200 and specifically the glass lid 212 allows entire length of tissue sample to be imaged efficiently. After imaging, the cassette 200 is removed from imaging device (e.g., SECM 100) and the glass lid 212 is opened. The lid 310 of the removable insert 210 is then closed. The lid 210 covers both the top region 302 and first opening 306 in the end of the removable insert 210.

The removable insert 210, following imaging is taken out of the cassette 200 and can be sent to lab for additional testing. Prior to shipment to the lab the entire removable insert 210 may be placed in ajar of preparation fluid such as formalin, as described above. The permeable material on the lid 310 of the removable insert allows the preparation fluids to reach the tissue samples without allowing the samples to pass out of the removable insert.

As is common, an individual patient may have multiple tissue samples acquired at the same time from the same or different lesions or tumors. The cassette 200, though generally disposable, may be used again with a new removable insert 210 for that patient. In this way for a single biopsy procedure where a number of biopsy samples are acquired, and a removable insert 210 is used for each, only a single cassette 200 may be required for that patient.

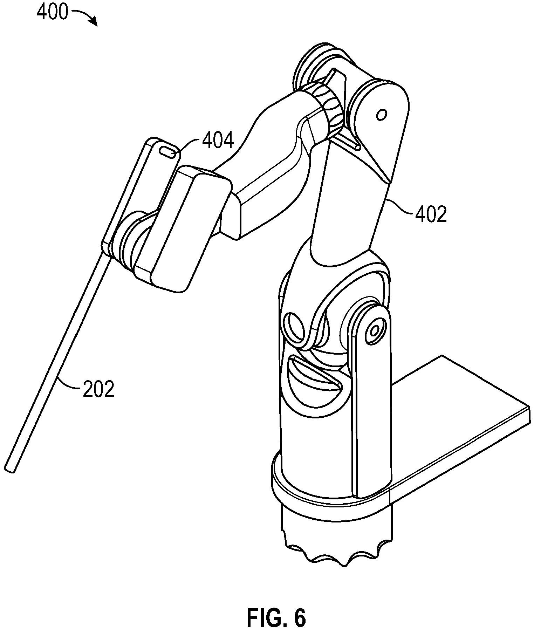

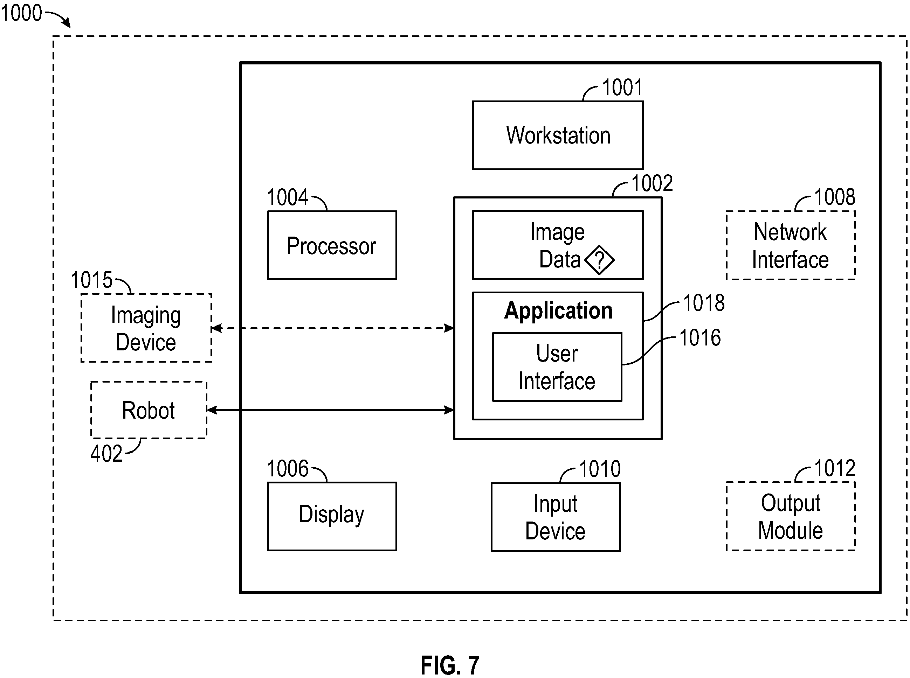

Those of skill in the art will recognize that the cassette 200 and removable insert 210 may be part of a robotic biopsy system 400 as depicted in Fig. 6. In such a robotic biopsy system 400 the biopsy device 202 is typically driven by a robotic arm 402. The robotic arm 402 may include the fluid source connection 404 to the biopsy device 202, and the robotic arm 402 may position the biopsy device 202 within a patient for collecting a tissue sample (e.g., a biopsy of a lesion or tumor). With reference to Fig. 7 a method 600 of utilizing a robotic arm 402 in preparation of a tissue sample for microscopy is presented. At step 602, the robotic arm, 402 after acquisition of the tissue sample may then be driven to insert the biopsy device 202 into the cassette 200, as shown in Fig. 2. The fluid source connection 404 on the robotic arm 402 at step 604 can inject fluid into the biopsy device 202 and expel the tissue sample from the biopsy device 202 and into the capture tube 208. The cassette 200 may be connected to a fluid source via the second channel 207 and at step 606 a pump may force fluid into second channel 207 and the capture tube 208 to force the tissue sample into the removable insert 210. At step 608 application of suction can be employed, if enabled, to drain the fluid from the removable insert 210. At step 610 the glass lid 212 can be closed by the robotic arm 402, and the cassette 200 can be grasped by the robotic arm 402 at step 612 to place the cassette 200 in the microscopy device 100 (a position as shown in Fig. 1) on the translation stage 118 for image acquisition at step 614. The acquired images may be communicated to a display in the operating room for review by an attending physician at step 616 and/or to a pathologist that is on call to perform initial ROSE examination of the images at step 618. After acquisition of the images, the removable insert 210 may be removed from the cassette 200 by the robotic arm 402 and placed in the formalin or other fixative solution for shipment to the laboratory for formal pathology examination.

Further extraction of biopsies can be undertaken by a similar process and be completely automated with respect to the procurement and handling of the tissue samples being biopsied. In this manner, all of the tissue samples can be contained in a single or a few fixation vessels and not require any direct handling by physicians or nurses. The removable inserts may be provided with a barcode or other machine-readable indicia to ensure that the tissue samples are at all times associated with the patient from whom they are taken. Similar indicia may also be placed on the fixation vessel so that a confirmation process can be undertaken at the laboratory.

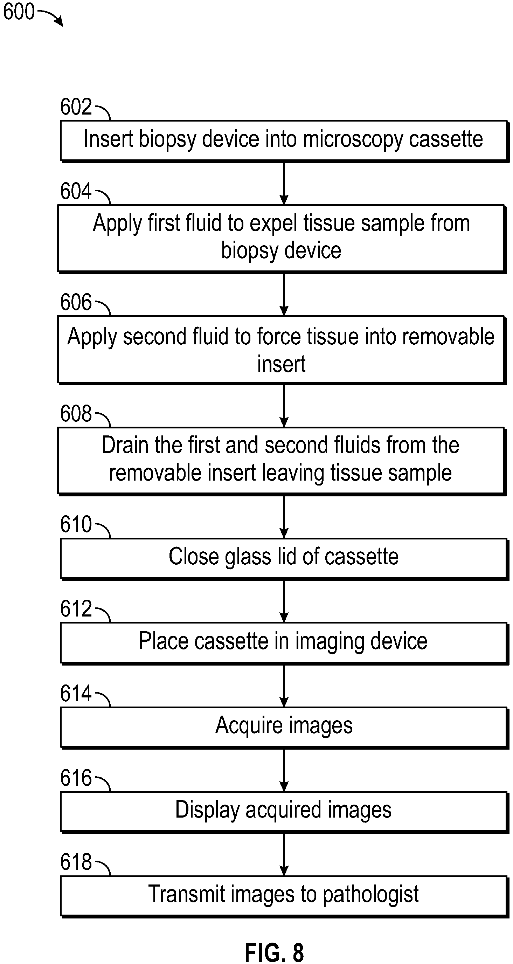

Still further, the images acquired by the microscopy system 100 may be analyzed by an artificial intelligence, neural network, or machine learning system that has been trained to recognize patterns related to the sufficiency of a biopsy sample, margin, and other features as well as to determine whether the collected sample has indicia of being cancerous. The machine learning may be a component of the application 1018 described below with reference to Fig. 8.

Reference is now made to Fig. 8, which is a schematic diagram of a system 1000 configured for use with the devices and systems of the disclosure. System 1000 may include a workstation 1001, and optionally connected to an imaging device (e.g., SECM system 100 (FIG. 1)). Workstation 1001 may be coupled with imaging device 1015, directly or indirectly, e.g., by wireless communication. Workstation 1001 may include a memory 1002, a processor 1004, a display 1006 and an input device 1010. Processor or hardware processor 1004 may include one or more hardware processors. Workstation 1001 may optionally include an output module 1012 and a network interface 1008. Memory 1002 may store an application 1018 and image data 1014. Application 1018 may include instructions executable by processor 1004 for executing the methods of the disclosure and operation of the imaging device 1015 and robot arm 402.

Application 1018 may further include a user interface 1016. Image data 1014 may include the microscopy scans including but not limited to SECM scans taken of biopsy samples. Processor 1004 may be coupled with memory 1002, display 1006, input device 1010, output module 1012, network interface 1008 and imaging device 1015. Workstation 1001 may be a stationary computing device, such as a personal computer, or a portable computing device such as a tablet computer. Workstation 1001 may embed a plurality of computer devices.

Memory 1002 may include any non-transitory computer-readable storage media for storing data and/or software including instructions that are executable by processor 1004 and which control the operation of workstation 1001 and, in some embodiments, may also control the operation of imaging device 1015. In an embodiment, memory 1002 may include one or more storage devices such as solid-state storage devices, e.g., flash memory chips. Alternatively, or in addition to the one or more solid-state storage devices, memory 1002 may include one or more mass storage devices connected to the processor 1004 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 1004. That is, computer readable storage media may include non-transitory, volatile and non-volatile, removable and non-removable media implemented in any method or technology for storage of information such as computer-readable instructions, data structures, program modules or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by workstation 1001.

Application 1018 may, when executed by processor 1004, cause display 1006 to present user interface 1016. User interface 1016 may be configured to present to the user a single screen including microscopy images of the tissue sample as well as other images and screens described herein. User interface 1016 may be further configured to display the images with different colors to enhance the resolution of aspects of the tissue depicted.

Network interface 1008 may be configured to connect to a network such as a local area network (LAN) consisting of a wired network and/or a wireless network, a wide area network (WAN), a wireless mobile network, a Bluetooth network, and/or the Internet. Network interface 1008 may be used to connect between workstation 1001 and imaging device 1015. Network interface 1008 may be also used to receive image data 1014. Input device 1010 may be any device by which a user may interact with workstation 1001, such as, for example, a mouse, keyboard, foot pedal, touch screen, and/or voice interface. Output module 1012 may include any connectivity port or bus, such as, for example, parallel ports, serial ports, universal serial busses (USB), or any other similar connectivity port known to those skilled in the art. Additional optic scans may be performed to visualize different depths within the tissue by adjusting the pin hole or focal point in the scanning microscope.

Application 1018 may have a variety of capabilities to enhance the utility of the images received from the imaging device 1015. For example, the application 1018 may stitch multiple the images from a single scan together to create a single image of the entire scanned portion of the tissue sample. Further, the application may control the depth of each scan to ensure the imaging at a desired depth for complete and accurate analysis.

The application 1018, may be in communication with and control aspects of the imaging device 1015, such as SECM system 100. The application may control adjustment of the SECM optical components such as the objective lens 110 to improve image. The application 1018 may include a calibration program to assess proper alignment of optical components of the imaging device 1015 and determine which adjustments need to be made. This may be assisted by markings on the glass lid 212 of the cassette 200. The markings provide a known size for reference that can be seen on final images.

While the application 1018 may automatically adjust the lens and mirror assemblies of an imaging device 1015 using motors connected to the components, alternatively, the application 1018 may presented on the display 1006 a user adjustment panel enabling a user to modify the component positions either to their liking or as recommended by the application 1018.

The application 1018 includes capabilities for evaluation of the quality of the image. This evaluation may include assessment of sharpness, completeness, and stain effectiveness, as well as others. As described above, where the standards for theses assessments are met the application may either display the microscopic image to the user on display 1006 or send it to a remote viewer for evaluation via a network interface 1008. Further, the application 1018 may include the ability to evaluate the images using artificial intelligence or machine learning to provide an analysis on the presence of cancerous cells, sufficiency of margin, and other aspects relevant to assessment of the tissue sample.

In instances where the application 1018 determines that an acquired image is not acceptable, the application 1018 can direct the imaging device 1015 to re-scan the tissue or for example using the translation stage 118 re-orient the tissue. Further, the display 1006 may present an indication that re-staining of the tissue sample is appropriate to achieve an acceptable image. This may be repeated until a satisfactory image is created.

As noted above, the network interface 1008 allows the application to directly transmit microscopic images acquired by the imaging device 1015 for pathology analysis. The images when sent for pathology analysis may include an indication of the original orientation of the tissue sample when the images were acquired. Accompanying the images may be the assessment of the AI or machine learning algorithms employed in the automatic analysis of application 1018. The network interface allows the attending physician to receive a pathology report in instances where such report can be quickly generated (e.g., using ROSE techniques). The report may include recommendations regarding any need for additional tissue samples to undertake a diagnosis or the diagnosis itself. The application 1018 may additionally be connected via the network interface to a central database (e.g., a cloud-based network storage) where the pathology report and images are stored and remain accessible to by the physician and pathologists.

While several aspects of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular aspects.

The invention may be described by reference to the following numbered paragraphs:
1. A microscopy cassette comprising: a first channel for receiving a biopsy device; a second channel in communication with a fluid source; a capture tube in communication with the first channel and the second channel; a recess configured to receive a removable insert and in fluid communication with the capture tube; and a glass lid covering the recess.
2. The microscopy cassette of paragraph 1, further comprising a reservoir in fluid communication with the recess.
3. The microscopy cassette of paragraph 2, further comprising a vacuum connection, wherein application of suction by the vacuum connection promotes movement of fluid from the recess to the reservoir.
4. The microscopy cassette of paragraph 1, further comprising a seal on the first channel configured to accept the biopsy device and prevent fluid from exiting the first channel.
5. The microscopy cassette of paragraph 1, further comprising a removable insert received in the recess.
6. The microscopy cassette of paragraph 5, wherein the removable insert is configured to receive a biopsy sample from the biopsy device.
7. The microscopy cassette of paragraph 6, wherein fluid from the biopsy device and from the second channel carries a tissue sample through the capture tube and into the removable insert.
8. A removable insert comprising: a top region, a bottom region, two ends and two angled side walls; an opening formed in one of the ends configured to receive a tissue sample; a mesh material formed on the bottom region and traversing an opening in the bottom region, the mesh material being liquid permeable but sized to retain a tissue sample received thereon; and a lid operably connected to one of the angled side walls, and movable from an open position to a closed position on the top region.
9. The removable insert of paragraph 8, further comprising a fluid permeable material on the lid permitting fluid to enter the removable insert when the lid is in the closed position.
10. The removable insert of paragraph 8, further comprising an end portion formed on the lid and covering the opening when the lid is in the closed position.
11. The removable insert of paragraph 8, wherein the top region, bottom region, two ends and two angled side walls are configured for receipt into a microscopy cassette.
12. The removable insert of paragraph 11, wherein the opening formed in one of the ends is configured to receive an end of a capture tube of the microscopy cassette.
13. A method of imaging a tissue sample comprising: receiving a biopsy device in a first channel of a microscopy cassette; injecting a first fluid into the biopsy device with a fluid to direct the tissue sample into a capture tube; injecting a second fluid into a second channel of the microscopy cassette; receiving the tissue sample in a removable insert; draining the first and second fluids from the removable insert; acquiring an image of the tissue sample.
14. The method of paragraph 13, further comprising closing a glass lid on the microscopy cassette.
15. The method of paragraph 13, further comprising applying a vacuum to assist in draining the first and second fluids from the removable insert.
16. The method of paragraph 14, further comprising translating the microscopy cassette and acquiring a plurality of images.
17. The method of paragraph 15, further comprising transmitting the acquired image to a display.
18. The method of paragraph 15, further comprising transmitting the acquired image to an application operating on a workstation, the application processing the image to make an initial pathology determination.
19. The method of paragraph 15, further comprising transmitting the acquired image to a laboratory for pathological examination.
20. The method of paragraph 13, wherein a robot inserts the biopsy device into the first channel of the microscopy cassette.

## Claims

1. A microscopy cassette comprising:
a first channel for receiving a biopsy device;
a second channel in communication with a fluid source;
a capture tube in communication with the first channel and the second channel;
a recess configured to receive a removable insert and in fluid communication with the capture tube; and
a glass lid covering the recess.

2. The microscopy cassette of claim 1, further comprising a reservoir in fluid communication with the recess.

3. The microscopy cassette of claim 2, further comprising a vacuum connection, wherein application of suction by the vacuum connection promotes movement of fluid from the recess to the reservoir.

4. The microscopy cassette of any preceding claim, further comprising a seal on the first channel configured to accept the biopsy device and prevent fluid from exiting the first channel.

5. The microscopy cassette of any preceding claim, further comprising a removable insert received in the recess.

6. The microscopy cassette of claim 5, wherein the removable insert is configured to receive a biopsy sample from the biopsy device; preferably wherein fluid from the biopsy device and from the second channel carries a tissue sample through the capture tube and into the removable insert.

7. A removable insert comprising:
a top region, a bottom region, two ends and two angled side walls;
an opening formed in one of the ends configured to receive a tissue sample;
a mesh material formed on the bottom region and traversing an opening in the bottom region, the mesh material being liquid permeable but sized to retain a tissue sample received thereon; and
a lid operably connected to one of the angled side walls, and movable from an open position to a closed position on the top region.

8. The removable insert of claim 7, further comprising a fluid permeable material on the lid permitting fluid to enter the removable insert when the lid is in the closed position; and/or further comprising an end portion formed on the lid and covering the opening when the lid is in the closed position.

9. The removable insert of claim 7 or claim 8, wherein the top region, bottom region, two ends and two angled side walls are configured for receipt into a microscopy cassette; preferably wherein the opening formed in one of the ends is configured to receive an end of a capture tube of the microscopy cassette.

10. A method of imaging a tissue sample comprising:
receiving a biopsy device in a first channel of a microscopy cassette;
injecting a first fluid into the biopsy device with a fluid to direct the tissue sample into a capture tube;
injecting a second fluid into a second channel of the microscopy cassette;
receiving the tissue sample in a removable insert;
draining the first and second fluids from the removable insert;
acquiring an image of the tissue sample.

11. The method of claim 10, further comprising closing a glass lid on the microscopy cassette and/or further comprising applying a vacuum to assist in draining the first and second fluids from the removable insert.

12. The method of claim 11, further comprising translating the microscopy cassette and acquiring a plurality of images; preferably further comprising transmitting the acquired image to a display.

13. The method of claim 12, further comprising transmitting the acquired image to an application operating on a workstation, the application processing the image to make an initial pathology determination.

14. The method of claim 13, further comprising transmitting the acquired image to a laboratory for pathological examination.

15. The method of claim 13 or claim 14, wherein a robot inserts the biopsy device into the first channel of the microscopy cassette.
